(19) Europäisches Patentamt
European Patent Office
Office européen des brevets

(11) Veröffentlichungsnummer: **0 013 254**
**B1**

(12) # EUROPÄISCHE PATENTSCHRIFT

(45) Veröffentlichungstag der Patentschrift: **04.08.82**

(21) Anmeldenummer: **79710015.3**

(22) Anmeldetag: **14.12.79**

(51) Int. Cl.³: **C 07 C 59/185**, C 07 C 59/205, C 07 C 51/373, C 07 C 69/716, C 07 C 67/333, C 07 C 121/34, C 07 C 120/00

(54) **Verfahren zur Herstellung von 5-Oxoalkansäuren, deren Estern und Nitrilen.**

(30) Priorität: **21.12.78 DE 2855195**

(43) Veröffentlichungstag der Anmeldung: **09.07.80 Patentblatt 80/14**

(45) Bekanntmachung des Hinweises auf die Patenterteilung **04.08.82 Patentblatt 82/31**

(84) Benannte Vertragsstaaten: **BE CH DE FR GB IT NL**

(56) Entgegenhaltungen: **CHEMICAL ABSTRACTS, Band 90, Nr. 11, 12. März 1979, Columbus, Ohio, US**

(73) Patentinhaber: **HOECHST AKTIENGESELLSCHAFT, Postfach 80 03 20, D-6230 Frankfurt/Main 80 (DE)**

(72) Erfinder: **Müller, Werner Heinrich, Dr., Taunusblick 5, D-6239 Eppstein/Taunus (DE)**
Erfinder: **Popp, Knut Ingemar, Hasenpfad 11, D-6232 Bad Soden am Taunus (DE)**

**EP 0 013 254 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen)

BUNDESDRUCKEREI BERLIN

# Verfahren zur Herstellung von 5-Oxoalkansäuren, deren Estern und Nitrilen

Es ist bekannt, daß man 5-Oxoalkansäuren durch aminkatalysierte Anlagerung eines Ketons, das zumindest ein Wasserstoffatom in α-Position zur Ketogruppe enthält, an eine α,β-ungesättigte Säure herstellen kann (DE-OS 2 348 536). Analog kann man 5-Oxoalkansäureester (DE-OS 2 325 160) bzw. 5-Oxoalkannitrile (DE-OS 2 329 923) aus den entsprechenden α,β-ungesättigten Carbonsäureestern bzw. Nitrilen herstellen.

Die 5-Oxoalkansäuren und deren Ester sind wichtige Ausgangsprodukte für Resorcine, welche bei der Gummi- und Holzleimherstellung oder als Antiseptika Verwendung finden. Die 5-Oxoalkannitrile können in die 5-Oxoalkansäuren bzw. -ester umgewandelt werden und sind außerdem Ausgangsmaterialien für α-Picoline, die zur Herstellung von Vinylpyridinen verwendet werden können (Comonomere bei der Copolymerisation mit Butadien, Styrol oder Acrylnitril).

Ein wesentlicher Nachteil der oben erwähnten Herstellverfahren beruht in ihrer mäßigen Produktivität.

Es bestand daher die Aufgabe, bei den obengenannten Verfahren die Raumzeitleistung zu erhöhen, ohne die Selektivität zu beeinträchtigen.

Es wurde nun ein Verfahren gefunden zur Herstellung von 5-Oxoalkansäuren, deren Estern oder Nitrilen, durch Umsetzung eines Ketons, das zumindest ein α-Wasserstoffatom enthält, bei erhöhter Temperatur mit einer α,β-ungesättigten Säure, deren Ester oder Nitril in der Flüssigphase in Gegenwart mindestens einer Verbindung aus der folgenden Gruppe als Katalysator: primäre Amine, primäre Aminoalkohole, primäre Aminocarbonsäuren, Schiffsche Basen aus primären Aminen und dem verwendeten Keton und, für den Fall der Umsetzung α,β-ungesättigter Carbonsäureester, die aus diesen mit primären Aminen entstehenden β-Aminopropionate, dadurch gekennzeichnet, daß man die Reaktion bei einem Druck oberhalb des Dampfdrucks des Reaktionsgemisches und unterhalb von 500 bar durchführt. Vorzugsweise bleibt man dabei unterhalb von 280 bar, insbesondere unterhalb von 200 bar.

Bisher war angegeben worden (DE-OS 2 348 536, DE-OS 2 325 160, DE-OS 2 329 923) der Druck sei nicht kritisch, man arbeite im allgemeinen zwischen Normaldruck und 50 Atmosphären, vorzugsweise bei dem der Reaktionstemperatur entsprechenden Dampfdruck. In DE-OS 2 246 284, DE-OS 2 355 859, DE-OS 2 738 388 war angegeben worden, der Druck sei nur so hoch zu wählen, daß das Reaktionsgemisch als Flüssigkeit anwesend ist.

Es war deshalb sehr überraschend, daß durch Erhöhung des Druckes über den der Reaktionstemperatur entsprechenden Dampfdruck der Reaktanten hinaus eine starke Erhöhung der Raumzeitleistung eintritt, obwohl man annehmen mußte, daß bei einer Flüssigphasenreaktion die Druckerhöhung keinen Einfluß haben sollte.

Im Lichte dieser Annahme ist auch die in Chemical Abstracts Band 90, Nr. 11 veröffentlichte Zusammenfassung der japanischen Anmeldung 78 124 219 zu interpretieren.

Gemäß dieser Zusammenfassung behandelt die japanische Anmeldung ein Verfahren zur Herstellung von Methyl-5-oxohexanoat und 5-Oxohexanonitril durch Erhitzen von Methylacrylat oder Acrylnitril zusammen mit einem Überschuß an Aceton in Gegenwart eines Amines wie z. B. Isopropylamin, Butylamin oder Cyclohexylamin und einer Lewis Säure. Beispielsweise wird in einem geschlossenen Rohr und bei einer Temperatur von 180°C gearbeitet. Dies bedeutete jedoch für den Fachmann zum Anmeldezeitpunkt der vorliegenden Anmeldung lediglich eine Arbeitsweise bei einem (der erhöhten Temperatur entsprechenden) erhöhten Dampfdruck, aber keineswegs bei einem Druck oberhalb des Dampfdrucks. Dies gilt deswegen, weil die Erzeugung und Beherrschung eines solchen höheren Drucks technisch aufwendig ist und davon keinerlei positive Effekte erwartet wurden.

Die günstigste Reaktionstemperatur hängt von der Art und Menge des verwendeten Ketons, der α,β-ungesättigten Verbindung und des verwendeten Katalysators ab. Im allgemeinen wird zwischen 100 und 250°C, vorzugsweise zwischen 150°C und der kritischen Temperatur der verwendeten Reaktionspartner gearbeitet.

Die Druckerhöhung über den Dampfdruck (bei der gewählten Reaktionstemperatur) der Reaktionskomponenten hinaus kann bewirkt werden durch den eigenen Flüssigkeitsdruck, der entsteht, wenn man den für die Umsetzung benutzten Reaktor bei Raumtemperatur mindestens so weitgehend mit den Reaktionskomponenten füllt, daß beim Erhitzen auf die Reaktionstemperatur die Gasphase verschwindet. Eine andere geeignete Methode, insbesondere bei höher siedenden Ketonen (mehr als 6 C-Atome) besteht darin, daß man ein Inertgas, wie z. B. $N_2$ oder Argon unter einem Druck in den Reaktor einpreßt, der den Dampfdruck der Reaktionskomponenten überschreitet.

Für die Umsetzung geeignete Ketone sind beispielsweise: Aceton, Methylethylketon, Diethylketon, Methylpropylketon, Methylisopropylketon, Methylbutylketon, Cyclopentanon, Cyclohexanon und Benzylmethylketon.

Als α,β-ungesättigte Verbindungen seien beispielsweise genannt: Acrylsäure, Methacrylsäure, Crotonsäure, Maleinsäure, Fumarsäure und Itaconsäure, sowie die entsprechenden Nitrile und die entsprechenden Alkylester mit bis zu 12, vorzugsweise bis zu 6 C-Atomen in der Alkylgruppe.

Als Katalysatoren sind beispielsweise geeignet:

Methylamin, Ethylamin, n-Propylamin, Isopropylamin, n-Butylamin, Isobutylamin, sek.Butylamin, tert. Butylamin, Amylamin, Isoamylamin, Ethylendiamin, Tetramethylendiamin, Cyclohexylamin, Cyclopentylamin, Benzylamin, Anilin, o-, m-, p-Toluidin, ε-Aminocapronsäure, Aminoethanol, Schiffsche Basen aus den als Ausgangsprodukte verwendeten Ketonen und den genannten primären Aminen, sowie N-substituierte β-Aminopropionate, welche sich aus den genannten Aminen im Falle des Einsatzes von α,β-ungesättigten Carbonsäureestern bilden.

Es ist von Vorteil, solche Katalysatoren zu verwenden, deren Siedepunkte unter dem Siedepunkt der gebildeten 5-Oxoverbindungen liegen. Beim erfindungsgemäßen Verfahren erhält man ein Reaktionsgemisch, welches beim Destillieren eine 1. Fraktion liefert, die unumgesetztes Keton, unumgesetzte α,β-ungesättigte Verbindung und den Katalysator enthält, 2. eine Hauptfraktion, welche hauptsächlich die gewünschte 5-Oxoverbindung enthält, sowie 3. einen Rückstand aus Verbindungen, die durch Addition von zwei Molekülen ungesättiger Verbindung an ein Ketonmolekül entstanden sind. Die 1. Fraktion kann ohne weitere Aufarbeitung in einem neuen Versuch wiederverwendet werden oder, bei kontinuierlicher Reaktionsführung, in den Reaktor zurückgeführt werden.

Die Menge des erfindungsgemäß zuzusetzenden Katalysators beträgt im allgemeinen zwischen 0,01 und 0,4 Mol pro Mol α,β-ungesättigter Verbindung.

Wird als Ausgangskomponente ein α,β-ungesättiger Carbonsäureester oder ein α,β-ungesättigtes Nitril verwendet, so ist der Zusatz einer sauren Verbindung zur Steigerung der Selektivität zweckmäßig, und zwar im allgemeinen in Mengen von ca. 0,01 bis 2 Gew.-%, vorzugsweise 0,1 bis 0,5 Gew.-%, bezogen auf das Reaktionsgemisch. Dabei können organische oder anorganische Säuren verwendet werden. Beispielsweise sind geeignet: Acrylsäure, Methacrylsäure, Crotonsäure, Benzoesäure, 2-Nitrobenzoesäure, 4-Nitrobenzoesäure, Hexansäure, p-Toluolsulfonsäure, 4-Methoxybenzoesäure, Essigsäure, Propionsäure, Äthylhexansäure, Borsäure, Salzsäure, Schwefelsäure und Phosphorsäure. Besonders vorteilhaft ist es bei der Addition eines Ketons an einen ungesättigten Ester, die dem Ester entsprechende ungesättigte Säure als Zusatz zu verwenden.

Das molare Verhältnis von Keton zu α,β-ungesättigter Verbindung beträgt im allgemeinen 1 : 2 bis 20 : 1, vorzugsweise 3 : 1 bis 8 : 1.

Die Umsetzung kann mit oder ohne Lösungs- oder Verdünnungsmittel ausgeführt werden.

Zweckmäßigerweise setzt man einen Polymerisationsinhibitor, wie z. B. Hydrochinon, Hydrochinonmonomethylether oder Phenothiazin zu.

Das erfindungsgemäße Verfahren kann bei diskontinuierlicher Arbeitsweise beispielsweise wie folgt betrieben werden: Die Reaktionskomponenten werden bei Raumtemperatur miteinander vermischt und in einen Reaktor, wie z. B. einen Autoklaven oder ein Schießrohr eingefüllt und für eine bestimmte Zeit auf die gewünschte Reaktionstemperatur erhitzt. Danach wird das Reaktionsgemisch schnell abgekühlt, analysiert und destillativ aufgearbeitet.

Bei kontinuierlicher Arbeitsweise kann das erfindungsgemäße Verfahren vorteilhaft wie folgt durchgeführt werden: Ein Gemisch bestehend z. B. aus Aceton, Methylacrylat, Polymerisationsinhibitor, einer Säure (wie z. B. Acrylsäure) und Isopropylamin wird mittels einer Dosierpumpe nach guter Durchmischung durch ein auf die gewünschte Reaktionstemperatur erhitztes Reaktionsrohr gepumpt. An dessen Ausgang ist eine automatische Stand- und Druckhaltung angebracht. Durch Inertgasüberlagerung wird der gewünschte Reaktionsdruck eingestellt.

Die folgenden Beispiele dienen zur Erläuterung der Erfindung.

## Herstellung von 5-Oxohexansäuremethylester (OHM)

### Beispiel 1

Ein Schießrohr von 30 ml Volumen wird je einmal mit 15, 18, 23 bzw. 27.5 ml des unten angegebenen Ausgangsgemisches gefüllt und 30 Minuten in ein 220°C heißes Ölbad versenkt. Dann wird abgekühlt und der Inhalt analysiert.

Die Versuchsbedingungen und Ergebnisse sind in Tabelle 1 zusammengefaßt.

Dabei bedeutet hier und in den folgenden Beispielen:

| | | |
|---|---|---|
| RZL (g/l · h) | = | Raumzeitleistung (Gramm pro Liter Reaktorvolumen und pro Stunde) |
| Sel/X | = | Mol Endprodukt pro Mol verbrauchtes Ausgangsmaterial X (Mol%) |
| Ac | = | Aceton |
| IPA | = | Isopropylamin |
| MAC | = | Methylacrylat |
| AS | = | Acrylsäure |
| HQ | = | Hydrochinon |
| Reaktorfüllung | = | Teil des Reaktorvolumens der bei Raumtemperatur mit dem Ausgangsgemisch gefüllt ist. |

| Ausgangsgemisch (Gew.-%) | | | | | |
|---|---|---|---|---|---|
| Ac | MAC | IPA | $H_2O$ | AS | HQ |
| 72,0 | 23,0 | 2,2 | 2,5 | 0,2 | 0,05 |

Tabelle 1

| Versuch | Reaktorfüllung | | Druck | Produktzusammensetzung (Gew.-%) | | | OHM | | |
|---|---|---|---|---|---|---|---|---|---|
| | (Vol.-%) | (g) | (bar) | Ac | MAC | OHM | Sel/MAC | Sel/Ac | RZL |
| 1 A | 50 | 12 | 40 | 67,6 | 16,4 | 7,9 | 72 | 73 | 63 |
| 1 B | 60 | 14,4 | 40 | 67,7 | 16,5 | 8,2 | 75 | 76 | 79 |
| 1 C | 77 | 18,4 | 60 | 65,4 | 12,4 | 13,4 | 82 | 82 | 167 |
| 1 D | 92 | 22,0 | 250 | 62,4 | 9,0 | 18,6 | 79 | 79 | 273 |

Die Versuche 1A und 1B sind Vergleichsversuche. Der Reaktionsdruck entspricht hierbei dem Dampfdruck des Reaktionsgemisches. Erhöhung der Reaktorfüllung von 50% (Versuch 1A) auf 77% (Versuch 1C) bzw. 92% (Versuch 1D) bewirkt eine Steigerung des Reaktordruckes von 40 bar (1A) auf 60 (1C) bzw. 250 bar (1D). Die Druckerhöhung von 40 auf 250 bar bewirkt eine Steigerung der OHM-Raumzeitleistung auf das 3.5fache (79 – 273 g/l · h).

## Beispiel 2

Ein 110-ml-Autoklav wird je einmal zu 50, 70, 80 bzw. 90% mit einem Ausgangsgemisch der unten angegebenen Zusammensetzung gefüllt und 45 Minuten auf 230°C erhitzt. Dann wird sofort gekühlt und der Inhalt analysiert. Tabelle 2 zeigt die Bedingungen und Ergebnisse dieser Versuche.

| Ausgangsgemisch (Gew.-%) | | | | | |
|---|---|---|---|---|---|
| Ac | MAC | IPA | BS | $H_2O$ | HQ |
| 75,8 | 20,0 | 1,0 | 0,1 | 3,0 | 0,05 |

BS = Benzoesäure.

Tabelle 2

| Versuch | Reaktorfüllung | | Druck | Produktzusammensetzung (Gew.-%) | | | OHM | | |
|---|---|---|---|---|---|---|---|---|---|
| | (Vol.-%) | (g) | (bar) | Ac | MAC | OHM | Sel/MAC | Sel/Ac | RZL |
| 2 A | 50 | 44 | 45 | 70,9 | 13,1 | 9,3 | 81 | 77 | 50 |
| 2 B | 70 | 61,6 | 76 | 69,5 | 10,6 | 12,4 | 79 | 79 | 83 |
| 2 C | 80 | 70,4 | 170 | 68,2 | 8,3 | 16,3 | 83 | 86 | 139 |
| 2 D | 90 | 79,2 | 250 | 64,9 | 4,2 | 21,1 | 80 | 78 | 203 |

Versuch 2A ist ein Vergleichsversuch beim Dampfdruck des Reaktionsgemisches.

Die Steigerung des Reaktordrucks von 45 bar (Versuch 2A) auf 250 bar (Versuch 2D) ergibt eine Steigerung der Raumzeitleistung von OHM auf das 4fache.

Beispiel 3

Vergleichsversuch 3A

Ein Strömungsrohr von 3.2 l Inhalt, an dessen Ende sich eine Stand- und Druckhaltung befinden, wird auf 230°C erhitzt. Sodann wird stündlich 3500 g Ausgangsmischung der unten angegebenen Zusammensetzung durch das Reaktionsrohr gepumpt. Der Dampfdruck beträgt 45 bar. Das Reaktionsprodukt wird am Reaktorende aufgefangen und analysiert. Die Ergebnisse sind in Tabelle 3 dargestellt.

Versuch 3B

Der Reaktordruck wird bei sonst genau gleichem Vorgehen mittels Inertgasüberlagerung auf 60 bar gesteigert. Die Ergebnisse sind in Tabelle 3 dargestellt.

| Ausgangsgemisch (Gew.-%) | | | | |
|---|---|---|---|---|
| Ac | MAC | IPA | $H_2O$ | AS |
| 80 | 15 | 3 | 2 | 0,1 |

Tabelle 3

| Versuch | Druck (bar) | Produktzusammensetzung (Gew.-%) Ac | MAC | OHM | RZL von OHM |
|---|---|---|---|---|---|
| 3 A | 45 | 71,1 | 4,2 | 13,4 | 147 |
| 3 B | 60 | 67,0 | 1,1 | 17,9 | 196 |

Eine Steigerung des Reaktionsdruckes von 45 auf 60 bar bewirkt also eine Erhöhung der Raumzeitleistung im kontinuierlichen Betrieb von 147 g/l · h auf 196 g/l · h.

Herstellung von 5-Oxohexannitril (OHN)

Beispiel 4

Ein 110-ml-Autoklav mit Manometer wird je einmal zu 60, 70 bzw. 90 Vol.-% (66 ml, 77 ml bzw. 99 ml) mit der unten angegebenen Ausgangsmischung gefüllt und dann jeweils 45 Minuten lang auf 230°C erhitzt. Daraufhin wird abgekühlt und das Reaktionsprodukt analysiert. Die Versuchsbedingungen und Ergebnisse sind in Tabelle 4 zusammengefaßt.

| Ausgangsgemisch (Gew.-%) | | | | | |
|---|---|---|---|---|---|
| IPA | Ac | AN | $H_2O$ | AS | HQ |
| 0,9 | 81,0 | 15,7 | 2,3 | 0,1 | 0,05 |

AN = Acrylnitril.

Tabelle 4

| Versuch | Druck (bar) | Reaktorfüllung (ml) | (g) | Produktzusammensetzung (Gew.-%) Ac | AN | OHN | OHN Sel/AN | Sel/Ac | RZL |
|---|---|---|---|---|---|---|---|---|---|
| 4 A | 44 | 66 | 52 | 72,8 | 8,4 | 12,2 | 80 | 78 | 77 |
| 4 B | 60 | 77 | 60 | 71,6 | 7,3 | 14,3 | 82 | 80 | 104 |
| 4 C | 280 | 99 | 78 | 68,4 | 4,7 | 19,1 | 83 | 79 | 181 |

Die Druckerhöhung von 44 bar im Vergleichsversuch 4A (Dampfdruck des Ausgangsgemisches bei 230° C) auf 280 bar (Versuch 4C) bewirkt also eine 2.4fache Erhöhung der Raumzeitleistung.

Herstellung von 5-Oxohexansäure (OHS)

Beispiel 5

Drei Schießrohre mit je 30 ml Inhalt werden mit 18 ml, 24 ml bzw. 27 ml der unten angegebenen Ausgangsmischung gefüllt und gemeinsam 30 Minuten in ein auf 230°C erwärmtes Ölbad getaucht. Dann wird abgekühlt und der Inhalt der Rohre analysiert. Die Versuchsbedingungen und Ergebnisse sind in Tabelle 5 zusammengefaßt.

| Ausgangsgemisch (Gew.-%) Ac | AS | IPA | MO | $H_2O$ | HQ |
|---|---|---|---|---|---|
| 68,9 | 24,0 | 1,0 | 5,0 | 1,0 | 0,05 |

MO = Mesityloxid.

Tabelle 5

| Versuch | Reaktorfüllung (Vol.-%) | (g) | Druck (bar) | Produktzusammensetzung (Gew.-%) Ac | AS | MO | OHS | OHS Sel/AS | Sel/Ac | RZL |
|---|---|---|---|---|---|---|---|---|---|---|
| 5 A | 60 | 15,7 | 40 | 63,4 | 16,1 | 5,4 | 9,7 | 68 | 79 | 102 |
| 5 B | 80 | 20,9 | 130 | 61,4 | 13,9 | 5,5 | 13,1 | 72 | 78 | 183 |
| 5 C | 90 | 23,5 | 250 | 59,7 | 11,6 | 5,6 | 17,1 | 76 | 83 | 268 |

Die Steigerung des Reaktionsdruckes von 40 bar (Vergleichsversuch 5A) auf 250 bar (Versuch 5C) bewirkt eine 2.6fache Erhöhung der Raumzeitleistung von OHS bei gleichzeitiger Steigerung der Selektivität von OHS bezogen auf AS und Aceton.

Herstellung von 5-Oxohexansäure-n-butylester (OHB)

Beispiel 6

Zwei 30 ml Schießrohre werden mit 18 bzw. 27 ml der unten angegebenen Ausgangsmischung gefüllt und gemeinsam 30 Minuten lang auf 230° C erhitzt. Dann wird gekühlt und der Inhalt der Rohre analysiert. Die Versuchsbedingungen und Ergebnisse sind in Tabelle 6 zusammengefaßt.

| Ausgangsgemisch (Gew.-%) | | | | | |
|---|---|---|---|---|---|
| Ac | BAC | $H_2O$ | AS | IPA | HQ |
| 71,4 | 22,0 | 2,5 | 0,12 | 1,0 | 0,05 |

BAC = n-Butylacrylat.

Tabelle 6

| Versuch | Reaktorfüllung (Vol.-%) | (g) | Druck (bar) | Produktzusammensetzung (Gew.-%) Ac | BAC | OHB | OHB Sel/BAC | Sel/Ac | RZL |
|---|---|---|---|---|---|---|---|---|---|
| 6 A | 60 | 14,1 | 40 | 67,2 | 13,5 | 10,8 | 87 | 80 | 102 |
| 6 B | 90 | 21,2 | 260 | 64,5 | 8,0 | 18,3 | 90 | 83 | 259 |

Die Druckerhöhung von 40 bar (Vergleichsversuch 6A) auf 260 bar (Versuch 6B) bewirkt eine 2.5fache Erhöhung der Raumzeitleistung von OHB.

Herstellung von 5-Oxohexansäure-ethylhexylester (OHEH)

Beispiel 7

Ein 110-ml-Autoklav wird zu 60 bzw. 90% mit einem Ausgangsgemisch der unten angegebenen Zusammensetzung gefüllt und 30 Minuten auf 230°C erhitzt. Dann wird sofort gekühlt und der Inhalt analysiert. Tabelle 7 zeigt die Versuchsbedingungen und die Ergebnisse.

| Ausgangsgemisch (Gew.-%) | | | | | |
|---|---|---|---|---|---|
| Ac | EHAC | IPA | $H_2O$ | AS | HQ |
| 74,4 | 22,0 | 1,5 | 2,0 | 0,1 | 0,05 |

EHAC = 2-Ethylhexylacrylat.

Tabelle 7

| Versuch | Reaktorfüllung (Vol.-%) | (g) | Druck (bar) | Produktzusammensetzung (Gew.-%) Ac | EHAC | OHEH | OHEH Sel/EHAC | Sel/Ac | RZL |
|---|---|---|---|---|---|---|---|---|---|
| 7 A | 60 | 53,4 | 41 | 68,9 | 5 | 18,6 | 83 | 82 | 180 |
| 7 B | 90 | 79,8 | 266 | 68,2 | 2,1 | 22,2 | 85 | 86 | 322 |

Kp von OHEH: 130°C (bei 7 mbar).

Durch die Druckerhöhung von 41 auf 266 bar steigt die Raumzeitleistung von 180 auf 322 g/l · h.

Herstellung von Acetonylbernsteinsäureester (AcBE)

Beispiel 8

Ein 110-ml-Autoklav wird zu 60% bzw. 90% mit einem Ausgangsgemisch der unten angegebenen Zusammensetzung gefüllt und 30 Minuten auf 200°C erhitzt. Dann wird sofort gekühlt und der Inhalt analysiert. Tabelle 8 zeigt die Versuchsbedingungen und Ergebnisse.

| Ausgangsgemisch (Gew.-%) | | | | |
|---|---|---|---|---|
| Ac | MDME | IPA | HQ | BS |
| 78,7 | 20,0 | 1,0 | 0,1 | 0,2 |

MDME = Maleinsäuredimethylester.

Tabelle 8

| Versuch | Reaktorfüllung (Vol.-%) | (g) | Druck (bar) | Produktzusammensetzung (Gew.-%) Ac | FDME | AcBE | ACBE Sel/MDME | Sel/Ac | RZL |
|---|---|---|---|---|---|---|---|---|---|
| 8 A | 60 | 54,4 | 28 | 74,4 | 11,4 | 8,7 | 72 | 75 | 86 |
| 8 B | 90 | 83,1 | 180 | 73,9 | 9,5 | 11,6 | 79 | 70 | 175 |

FDME = Fumarsäuredimethylester (unter Reaktionsbedingungen findet Isomerisierung statt).
AcBE = Acetonylbernsteinsäuredimethylester.

$$H_3CCOCH_2CH-COOCH_3 \quad (CH-CH_2COOCH_3) \qquad Kp_3\ 122-123°C$$

Durch die Druckerhöhung über den Dampfdruck des Reaktionsgemisches von 28 bar hinaus (Vergleichsversuch 8A) auf 180 bar (Versuch 8B) steigt die Raumzeitleistung von AcBE von 86 auf 175 g/l · h.

Herstellung von 4-Methyl-5-Oxohexansäuremethylester (MOHM) und 5-Oxoheptansäuremethylester (OHPM)

Beispiel 9

Ein 110-ml-Autoklav wird zu 60 bzw. 90% mit einem Ausgangsgemisch der unten angegebenen Zusammensetzung gefüllt und 30 Minuten auf 230°C erhitzt. Dann wird gekühlt und der Inhalt analysiert. Tabelle 9 zeigt die Versuchsbedingungen und Ergebnisse.

| Ausgangsgemisch (Gew.-%) | | | | |
|---|---|---|---|---|
| MEK | MAC | IPA | AS | HQ |
| 78,3 | 20,0 | 1,5 | 0,1 | 0,05 |

Tabelle 9

| Versuch | Reaktorfüllung (Vol.-%) | (g) | Druck (bar) | Produktzusammensetzung (Gew.-%) MEK | MAC | MOHM | OHPM | MOHM + OHPM Sel/MAC | Sel/MEK | RZL |
|---|---|---|---|---|---|---|---|---|---|---|
| 9 A | 60 | 53,5 | 30 | 68,3 | 8 | 14,7 | 3,0 | 80 | 78 | 172 |
| 9 B | 90 | 80,8 | 180 | 63,5 | 2,8 | 22,0 | 4,5 | 84 | 81 | 390 |

**Durch die Erhöhung des Druckes über den Dampfdruck (30 bar) des Reaktionsgemisches hinaus auf 180 bar steigt die Raumzeitleistung von 172 auf 390 g/l · h.**

Herstellung von 3-(2-Oxo-cyclohexyl)-propansäuremethylester (OCPM)

Beispiel 10

Ein 110-ml-Autoklav wird in einem ersten Versuch (10A) zu 90% mit einem Ausgangsgemisch der unten angegebenen Zusammensetzung gefüllt und 30 Minuten lang auf 180°C erhitzt. Dann wird schnell gekühlt und der Inhalt analysiert. In einem zweiten Versuch (10B) werden zusätzlich vor dem Aufheizen 150 bar $N_2$ zugegeben, ansonsten wird genauso verfahren wie in Versuch 10A. Die Versuchsbedingungen und Ergebnisse sind in Tabelle 10 zusammengefaßt.

| Ausgangsgemisch (Gew.-%) | | | | |
|---|---|---|---|---|
| Cyclohexanon | MAC | IPA | BS | HQ |
| 63,0 | 36,0 | 0,6 | 0,2 | 0,2 |

Tabelle 10

| Versuch | Reaktorfüllung (Vol.-%) | (g) | Druck (bar) | Produktzusammensetzung (Gew.-%) CHO | MAC | OCPM | OCPM Sel/MAC | Sel/CHO | RZL |
|---|---|---|---|---|---|---|---|---|---|
| 10 A | 90 | 92,5 | 6 | 51,5 | 24,7 | 20,0 | 83 | 90 | 336 |
| 10 B | 90 | 92,5 | 280 | 47,4 | 21,1 | 26,8 | 84 | 92 | 450 |

HO = Cyclohexanon

Dieses Beispiel zeigt, daß bei einem höhersiedenden Keton wie Cyclohexanon die Erhöhung der Raumzeitleistung durch Einpressen von Stickstoff in den Reaktor erreicht wird.

**Patentansprüche**

1. Verfahren zur Herstellung von 5-Oxoalkansäuren, deren Estern oder Nitrilen, durch Umsetzung eines Ketons, das zumindest ein α-Wasserstoffatom enthält, bei erhöhter Temperatur mit einer α,β-ungesättigten Säure, deren Ester oder Nitril, in der Flüssigphase in Gegenwart mindestens einer Verbindung aus der folgenden Gruppe als Katalysator: primäre Amine, primäre Aminoalkohole, primäre Aminocarbonsäuren, Schiffsche Basen aus primären Aminen und dem verwendeten Keton und, für den Fall der Umsetzung α,β-ungesättigter Carbonsäureester, die aus diesen mit primären Aminen entstehenden β-Aminopropionate, dadurch gekennzeichnet, daß man die Reaktion bei einem Druck oberhalb des Dampfdrucks des Reaktionsgemisches und unterhalb von 500 bar durchführt.

2. Verfahren nach Anspruch 1, dadurch gekennzeichnet, daß man den Druck derart erzeugt, daß man den für die Umsetzung benutzten Reaktor bei Raumtemperatur mindestens so weitgehend mit dem Ausgangsgemisch füllt, daß beim Erhitzen auf die Reaktionstemperatur die Gasphase verschwindet.

3. Verfahren nach Anspruch 1, dadurch gekennzeichnet, daß man den Druck derart erzeugt, daß man ein Inertgas unter einem Druck in den für die Umsetzung benutzten Reaktor einpreßt, der den Dampfdruck der Reaktionskomponenten überschreitet.

4. Verfahren nach einem der Ansprüche 1 bis 3, dadurch gekennzeichnet, daß man die Reaktion bei einem Druck unterhalb von 280 bar durchführt.

5. Verfahren nach einem der Ansprüche 1 bis 3, dadurch gekennzeichnet, daß man die Reaktion bei einem Druck unterhalb von 200 bar durchführt.

**Claims**

1. A process for the manufacture of 5-oxoalkanoic acids, their esters or nitrils, by reaction of a ketone containing at least one α-hydrogen atom, at elevated temperature, with an α,β-unsaturated acid, its ester or nitril, in the liquid phase, in the presence of at least one compound selected from the group consisting of: primary amines, primary aminoalcohols, primary aminocarboxylic acids, Schiff's bases of primary amines and the ketone used, and, if α,β-unsaturated carboxylic acid esters are reacted, the β-aminopropionates formed from the latter esters and primary amines, characterized by conducting

the reaction under a pressure above the vapor pressure of the reaction mixture and below 500 bars.

2. The process as claimed in claim 1, characterized by producing the pressure by charging the reactor used for the reaction, at room temperature, at least to such a degree with the starting mixture that the gaseous phase vanishes on heating to the reaction temperature.

3. The process as claimed in claim 1, characterized by producing the pressure by pressing into the reactor used for the reaction an inert gas under a pressure exceeding the vapor pressure of the reaction components.

4. The process as claimed in any one of claims 1 to 3, characterized by carrying out the reaction under a pressure below 280 bars.

5. The process as claimed in any one of claims 1 to 3, characterized by carrying out the reaction under a pressure below 200 bars.

**Revendications**

1. Procédé de préparation d'acides oxo-5 alcanoïques, de leurs esters ou de leurs nitriles, par réaction d'une cétone contenant au moins un atome d'hydrogène en $\alpha$, à température élevée, avec un acide insaturé en $\alpha,\beta$, l'un de ses esters ou son nitrile, en phase liquide, en présence d'au moins un composé à action catalytique pris dans l'ensemble constitué par les amines primaires, les amino-alcools primaires, les acides amino-carboxyliques primaires, les bases Schiff dérivant d'amines primaires et de la cétone utilisée, et, dans le cas de la réaction d'esters d'acides carboxyliques insaturés en $\alpha,\beta$, les $\beta$-amino-propionates formés à partir de ces esters et d'amines primaires, procédé caractérisé en ce qu'on effectue la réaction sous une pression supérieure à la pression de vapeur du mélange réactionnel et inférieure à 500 bar.

2. Procédé selon la revendication 1 caractérisé en ce qu'on crée la pression en introduisant le mélange réactionnel dans le récipient devant servir à la réaction de manière à atteindre un taux de remplissage de celui-ci, à la température ambiante, au moins assez élevé pour que la phase gazeuse disparaisse lors du chauffage à la température réactionnelle.

3. Procédé selon la revendication 1, caractérisé en ce qu'on crée la pression en introduisant, dans le récipient utilisé pour la réaction, un gaz inerte sous une pression supérieure à la pression de vapeur des composantes réactionnelles.

4. Procédé selon l'une quelconque des revendications 1 à 3, caractérisé en ce qu'on effectue la réaction sous une pression inférieure à 280 bar.

5. Procédé selon l'une quelconque des revendications 1 à 3, caractérisé en ce qu'on effectue la réaction sous une pression inférieure à 200 bar.